# EUROPÄISCHE PATENTSCHRIFT

(11) **EP 0 515 883 B1**
(45) Veröffentlichungstag und Bekanntmachung des Hinweises auf die Patenterteilung: **03.05.1995**
(21) Anmeldenummer: 92107848.1
(22) Anmeldetag: 08.05.1992
(51) Int. Cl.: B01L 3/02, B01L 3/14, G01N 33/48

(54) **Einrichtung zur sicheren Entnahme von Blut aus einem Vorratsgefäss**
Device for safe sampling of blood from a container
Appareil pour prélever sans risque le sang d'un récipient

(30) Priorität: 28.05.1991 DE 4117483; 26.03.1992 DE 4209872
(43) Veröffentlichungstag der Anmeldung: 02.12.1992
(73) Patentinhaber: DADE INTERNATIONAL INC., Deerfield, Illinois 60015-0778 (US)
(72) Erfinder: Kratzer, Michael, Dr., D-80802 München (DE); Freiherr von der Goltz, Volker, D-83370 Seeon (DE)
(74) Vertreter: Haft, von Puttkamer, Berngruber, Czybulka

(56) Entgegenhaltungen:
- EP-A- 0 223 244
- EP-A- 0 316 599
- EP-A- 0 339 429
- EP-A- 0 347 837
- GB-A- 1 234 044
- US-A- 4 463 616

## Beschreibung

Die Erfindung betrifft eine Einrichtung zur sicheren Entnahme von Blut aus einem Vorratsgefäß nach dem Oberbegriff des Patentanspruches 1.

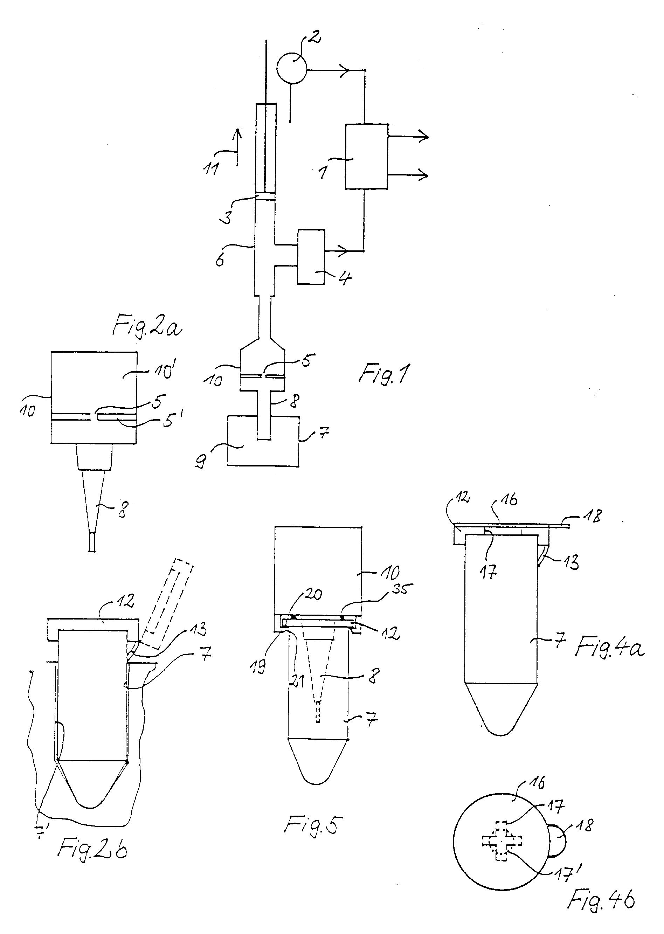

Es ist bekannt, gemäß Figur 1 die Blutungszeit in-vitro dadurch zu messen, daß Blut 9 aus einem Vorratsgefäß 7 über eine Apertur 5 in einen Zylinder 6 dadurch eingesaugt wird, daß im Zylinder 6 ein Kolben 3 durch einen Schrittmotor 2 in der Richtung 11 bewegt wird. Ein Drucksensor 4 mißt dabei den Druck, der in dem dem Kolben 3 vorgelagerten Raum herrscht. Dieser Druck wird dadurch auf einen konstanten Wert gehalten, daß ein Prozessor 1 den Schrittmotor 2 in Abhängigkeit vom Signal des Drucksensors 4 ansteuert. Aus der Bewegung des Kolbens und dem Durchmesser des Zylinders 6 errechnet der Prozessor 1 den Volumenstrom des Blutes durch die Apertur 5. Die Apertur 5, deren Durchmesser beispielsweise bei etwa 150 »m liegt, bildet einen verletzten Teil einer Arteriole nach. Sie befindet sich beispielsweise in einem Zelluloseacetat-Filter, der mit Kollagen beschichtet ist. Der Filter wird vor der Messung mit ADP getränkt. Nach dem beschriebenen Verfahren ist eine reproduzierbare Messung der Blutungszeit in vitro und des Blutungsvolumens möglich.

Derartige Messungen werden in einer Meßeinrichtung dadurch ausgeführt, daß ein Vorratsgefäß für Blut in die Meßeinrichtung in einer vorbestimmten Position manuell eingesetzt wird, der Verschluß des Vorratsgefäßes vor Ausführung der Messung geöffnet wird, in das geöffnete Gefäß die mit einem Aperturhalter verbundene Kapillare eingesetzt wird und ein mit dem Zylinder verbundener Meßkopf mit der der Kapillare abgewandten Seite des Adapterhalters dicht verbunden wird.

Ein Problem bei derartigen Messungen kann darin bestehen, daß bei der Handhabung des Vorratsgefäßes und beim Einsetzen der Kapillare in das Vorratsgefäß Kontakte zu dem in dem Vorratsgefäß befindlichen Blut entstehen können, die insbesondere im Hinblick auf die Übertragung von Aids oder Hepatitis unbedingt vermieden werden müssen. Kontakte zu dem Blut können auch dann entstehen, wenn nach der Ausführung der Messung die Kapillare aus dem Vorratsgefäß herausgenommen wird.

Die Aufgabe der vorliegenden Erfindung besteht darin, eine Einrichtung zur sicheren Handhabung eines Vorratsgefäßes für Blut anzugeben, mit deren Hilfe Kontakte zu dem in dem Vorratsgefäß enthaltenen Blut sowohl beim Öffnen des Vorratsgefäßes als auch beim Einführen einer Kapillare in das Vorratsgefäß vermeidbar sind.

Diese Aufgabe wird durch eine Einrichtung der eingangs genannten Art gelöst, die durch die in dem kennzeichnenden Teil des Patentanspruches 1 angegebenen Merkmale gekennzeichnet ist.

Der wesentliche Vorteil der vorliegenden Erfindung besteht darin, daß bei der Bestimmung der Blutungszeit in-vitro, bei der einem Vorratsgefäß Blut über eine Kapillare entnommen wird, die Möglichkeit von Kontakten zu dem im Vorratsgefäß befindlichen Blut weitgehendst vermieden wird. Dadurch kann die Übertragung von äußerst gefährlichen Krankheiten, wie z.B. Aids oder Hepatitis vermieden werden. Dies ist bei der Vornahme von Messungen in Labors bzw. in Krankenhäusern in größeren Umfang von Vorteil, weil das die Messung vornehmende Personal geschützt wird und weil infolge der automatisch gegebenen Sicherheit die Arbeiten zur Einsparung von Arbeitszeit einfach und schnell durchgeführt werden können.

Vorteilhafterweise lassen sich Kontakte zum Blut des Vorratsgefäßes sowohl beim Einführen der mit dem Aperturhalter verbundenen Kapillare in das Vorratsgefäß als auch beim Entfernen der Kapillare aus dem Vorratsgefäß vermeiden.

Bei einer bevorzugten Ausführungsform ist der Adapterhalter an der dem Meßkopf zugewandten Seite so verschlossen, daß auch ein Austreten von Blut aus dem Vorratsraum des Adapterhalters nicht erfolgen kann.

Weitere bevorzugte Ausgestaltungen der Erfindung gehen aus den Unteransprüchen hervor.

Im folgenden werden die Erfindung und deren Ausgestaltungen im Zusammenhang mit den Figuren näher erläutert. Es zeigen:
- Fig. 1: eine schematische Darstellung einer bekannten Meßeinrichtung zur Erläuterung der Handhabung des Vorratsgefäßes und der mit dem Aperturhalter verbundenen Kapillare;
- Fig. 2: die mit dem Aperturhalter verbundene Kapillare sowie ein darunter befindliches Vorratsgefäß, das durch einen Deckel verschlossen ist;
- Fig. 3 a, b: eine Seitenansicht einer ersten Ausführungsform des Vorratsgefäßes bzw. eine Aufsicht auf den Deckel dieses Vorratsgefäßes;
- Fig. 4 a, b: eine Seitenansicht einer zweiten Ausführungsform des Vorratsgefäßes sowie eine Aufsicht auf den Deckel dieses Vorratsgefäßes;
- Fig. 5: eine erste Art der Verriegelung des Vorratsgefäßes am Aperturhalter;
- Fig. 6: eine zweite Art der Verriegelung des Vorratsgefäßes am Aperturhalter;
- Fig. 7: eine weitere Ausführungsform der Erfindung;
- Fig. 8: einen Aperturhalter, dessen dem Meßkopf zugewandte Seite durch eine eine Öffnung aufweisende Folie verschlossen ist;
- Fig. 9, 10: Vorratsgefäße mit mehreren Teilräumen;
- Fig. 11: das Vorratsgefäß der Figur 9 mit einem Aperturhalter, der in zwei Blutaufnahmeräume unterteilt ist, und
- Fig. 12: einen Einsatzclip zur Verkleinerung des Volumens eines Vorratsgefäßes.

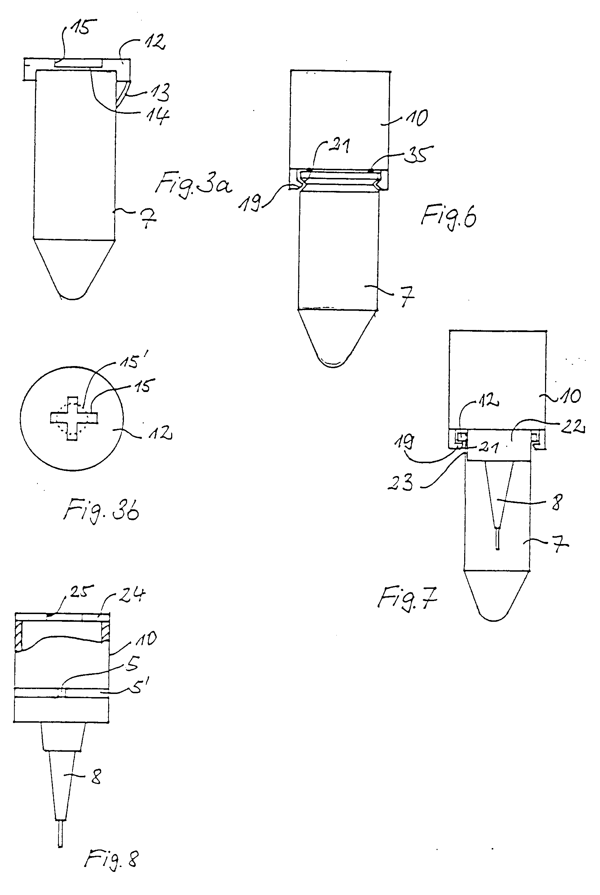

Aus der Figur 2 ist ersichtlich, daß der Aperturhalter 10 zusammen mit der Kapillare 8 vorzugsweise einteilig als ein Wegwerfteil aus Kunststoff ausgebildet ist. In dem Adapterhalter 10 wird, wie dies schematisch dargestellt ist, ein die Apertur 5 aufweisendes Teil 5' derart gehalten, daß durch die Kapillare 8 eingesaugtes Blut der Apertur 5 zugeführt wird. Über dem Teil 5' befindet sich ein das durch die Apertur 5 hindurchgetretene Blut aufnehmender Vorratsraum 10'. Als Teil 5' dient beispielsweise ein mit Kollagen beschichtetes Zelluloseacetat-Filter.

In der aus der Figur 2 ersichtlichen Weise weist das Vorratsgefäß 7 vorzugsweise die Form eines an einem Ende geschlossenen zylindrischen Gefäßes auf, dessen anderes Ende durch ein Deckelteil 12 dicht verschließbar ist. Vorzugsweise ist das Deckelteil 12 mit dem Vorratsgefäß 7, die beide aus einem Kunststoffmaterial bestehen, über ein sogenanntes Filmscharnier 13 einstückig verbunden, so daß das Deckelteil 12 unverlierbar am Vorratsgefäß 7 gehalten wird. Bei der Handhabung eines mit Blut 9 gefüllten Vorratsgefäßes 7 wird so vorgegangen, daß das Vorratsgefäß 7 in eine Aufnahmemulde bzw. -hülse 7' unterhalb des Meßkopfes der Meßeinrichtung eingesetzt wird. Bei diesem Vorgang wird das Vorratsgefäß 7 in Bezug auf den Meßkopf justiert. Vor dem Einsetzen wird das Vorratsgefäß 7 zur Durchmischung des Blutes 9 mehrfach invertiert, danach wird das Blut etwa 4 Minuten lang inkubiert, wobei es auf etwa 37 °C erhitzt wird, und nochmals invertiert. Nach dem Einsetzen in die Aufnahmehülse 7' wird normalerweise das Deckelteil 12 manuell geöffnet und die Kanüle 8 wird in das Vorratsgefäß 7 eingesetzt. Danach wird von oben der Meßkopf gegen den Aperturhalter 10 gedrückt.

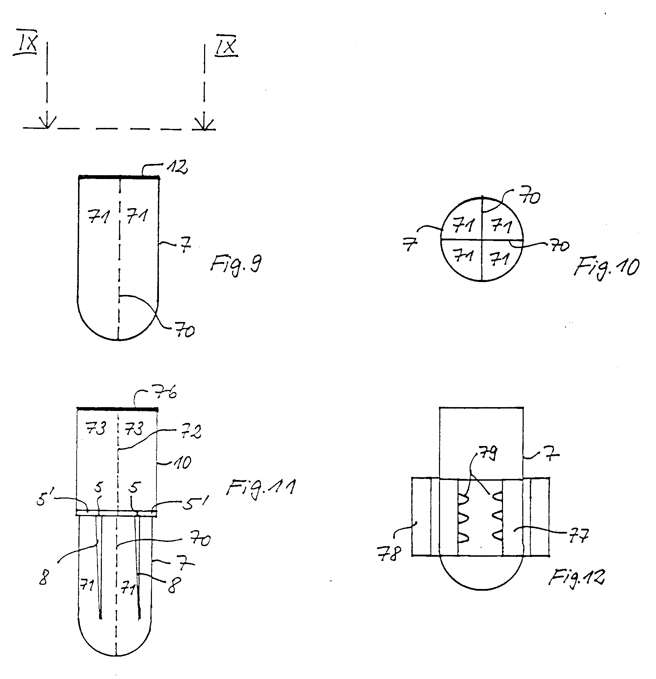

Um bei diesem Vorgang die Gefahr eines Kontaktes zu dem im Vorratsgefäß befindlichen Blut 9 zu vermeiden, wird das Deckelteil 12 des Vorratsgefäßes 7 so ausgebildet, daß die Kapillare 8 in das Vorratsgefäß 7 eingeführt werden kann, ohne daß das Deckelteil 12 geöffnet werden muß. Zu diesem Zweck kann das Deckelteil 12 gemäß Figur 3 a, b zumindest in einem Teilbereich die Form einer Schwachstelle oder einer dünnen Membran 14 aufweisen, die durch ein Spezialwerkzeug oder durch die Spitze der Kapillare 8 durchstoßen werden kann. Vorzugsweise weist die genannte Schwachstelle 14 die Form eines Kreuzschlitzes 15 oder eines zentralen Bereiches 15' auf, der besonders leicht durchstoßbar ist. Auf diese Weise wird vermieden, daß nach dem Einsetzen des Vorratsgefäßes 7 in die Aufnahmehülse 7' das Deckelteil 12 geöffnet werden muß.

Gemäß Figur 4 a, b kann das Deckelteil 12 auch eine durch eine Folie, zweckmäßigerweise eine Adhäsionsfolie 16 oder dergleichen, verschlossene Öffnung 17 aufweisen, wobei die Adhäsionsfolie 16 vorzugsweise auf der Außenseite des Deckelteiles 12 über der Öffnung 17 angeordnet ist. Vorzugsweise weist die Adhäsionsfolie 16 einen Griffbereich 18 auf, der über den Rand des Deckelteiles 12 vorsteht, um ein erleichtertes Abnehmen der Adhäsionsfolie 16 zu ermöglichen.

Die Öffnung 17 kann in der aus der Figur 4 b ersichtlichen Weise die Form eines Kreuzschlitzes aufweisen. Beispielsweise kann die Öffnung 17 jedoch auch, wie dies in der Figur 4b durch eine gepunktete Linie dargestellt ist, die Form eines zentralen Loches besitzen.

Zur weiteren Erhöhung der Sicherheit der Handhabung des Vorratsgefäßes 7 und des Adapterhalters 10 mit der Kapillare 8 weisen sowohl das Vorratsgefäß 7 als auch der Aperturhalter 10 an den aneinander zugewandten Bereichen Verriegelungseinrichtungen auf, durch die bewirkt wird, daß das Vorratsgefäß 7 nach dem Einführen der Kapillare 8 durch das Deckelteil 12 aneinander verriegelt werden, wenn beispielsweise der Meßkopf der Meßeinrichtung den Aperturhalter 10 gegen das Vorratgefäß 7 drückt. Hierzu weist der Aperturhalter 10 an seinem dem Vorratsgefäß 7 zugewandten Seite beispielsweise mehrere über seinen Umfang verteilte Verriegelungsvorsprünge 19 auf, die eine Verriegelungsschulter 20 hintergreifen, die durch den Übergang zwischen dem auf das Vorratsgefäß 7 aufgesetzten Deckelteil 12 und dem Umfang des Vorratsgefäßes 7 gebildet ist (Fig. 5). Um ein besonders leichtes Ausfedern der Verriegelungsvorsprünge 19 zu erreichen, können diese an der dem Vorratsgefäß 7 zugewandten Seite sogenannte Einfahrschrägen 21 aufweisen, durch die bewirkt wird, daß die Verriegelungsvorsprünge 19 federnd nach außen gedrückt werden, wenn das Deckelteil 12 an ihnen angreift. Derartige Einfahrschrägen können auch am oberen Rand des Deckelteiles 12 vorgesehen sein.

Gemäß Figur 6 können die Verriegelungseinrichtungen allgemein ausgedrückt aus einem Verriegelungsvorsprung 19 und einer Verriegelungsvertiefung 21 bestehen, wobei der Verriegelungsvorsprung 19 an dem Aperturhalter 10 (oder dem Vorratsgefäß bzw. dem Deckelteil) und die Verrieglungsvertiefung 21 am Vorratsgefäß 7 bzw. am Deckelteil 12 (oder am Aperturhalter) vorgesehen sind.

Besonders bevorzugt ist eine Ausführungsform, bei dem der Aperturhalter 10 gemäß Figur 7 einen stöpselartigen Verschlußbereich 22 aufweist, der beim Aufbringen des Vorratsgefäßes 7 am Aperturhalter 10 in den oberen Endbereich des Vorratsgefäßes 7 einfährt, um diesen zu verschließen, so daß auch bei einer Schräglage kein Blut austreten kann. Um bei der Meßoperation und der Blutentnahme das Eintreten von Luft in das Vorratsgefäß 7 zu ermöglichen, ist dafür Sorge getragen, daß zwischen dem Verschlußbereich 22 und der Innenwandung des Vorratsgefäßes 7 ein sich wenigstens über einen Teilbereich des Umfanges des Vorratsgefäßes 7 und des Verschlußbereiches 22 erstreckender Belüftungsspalt 23 vorgesehen ist, über den das Innere des Vorratsgefäßes 7 mit der Außenluft in Verbindung steht. Der Belüftungsspalt 7 ist vorzugsweise so bemessen, daß kein Blut durch ihn hindurchtreten kann, da in den Belüftungsspalt eintretendes Blut zur Verstopfung desselben führt. Beispielsweise liegt dieser Belüftungsspalt in der Größenordnung von 100 »m.

In diesem Fall ist das Deckelteil 12 des Vorratsgefäßes 7 vorzugsweise gemäß Figur 4 ausgestaltet, wobei die zentrale Öffnung (gepunktete Linie in Figur 4b) so bemessen ist, daß ihre Randbereiche nicht über die Randbereiche des Vorratsgefäßes 7 nach Innen ragen. Im Falle eines Vorratsgefäßes 7 mit einem kreisförmigen Durchmesser ist der Durchmesser der zentralen Öffnung des Deckelteiles 12 geringfügig größer als der Innendurchmesser des Vorratsgefäßes 7, so daß der Verschlußbereich 22 nach dem Abziehen des Adhäsionsfolie 18 ohne weiteres in das Vorratsgefäß 7 einführbar ist.

Gemäß Figur 8 kann auch das dem Prüfkopf zugewandte Ende des Aperturhalters 10 durch ein Teil 24 verschlossen sein, in dem sich ein mittiges Loch 25 befindet, durch das beispielsweise ADP in den Aperturhalter 10 eingebbar ist, so daß es vom porösen Material eines die Apertur 5 aufweisenden, in dem Aperturhalter 10 gehaltenen Teiles 5' eindringen kann. Das Teil 24 weist vorzugsweise die Form einer mit dem Aperturhalter 10 verbundenen Adhäsionsfolie auf. Durch die Membran 24 wird verhindert, daß bei der Meßoperation durch die Apertur 5 hindurchgetretenes Blut aus dem Aperturhalter 10 bei einer Schräglage desselben austritt, die bei der Entnahme des Aperturhalters 10 und des vorzugsweise mit diesem verbundenen Vorratsgefäßes 7 auftreten könnte.

Die Folie 16 der Figur 4 a, b kann einen extrem kleinen Adhäsionsfaktor aufweisen, so daß beim Abziehen an der Folie kein Blut hängen bleibt. Es ist denkbar, zwischen dem Aperturhalter 10 und dem Vorratsgefäß 7 ein Dichtungselement 35 vorzusehen (Figuren 5, 6) durch das eine dichte Verbindung der Teile 10 und 7 ermöglicht wird.

Wie dies aus den Figuren 9 und 10 ersichtlich ist, kann das Vorratsgefäß 7 durch eine oder mehrere Trennwände 70 in zwei oder mehrere Teilräume 71 unterteilt werden. Dabei ist dafür Sorge getragen, daß sich die Trennwände 70 zur offenen Seite des Gefäßes hin ebenso hoch erstrecken, wie die Wandung des Vorratsgefäßes 7. Dabei ist das das Vorratsgefäß 7 verschließende Deckelteil 12 so gestaltet, daß es für jeden Teilraum 71 einen Bereich aufweist, durch den hindurch eine Kapillare in den entsprechenden Teilraum 71 einführbar ist. Dabei kann der Bereich in der zuvor beschriebenen Weise ausgestaltet sein.

Es ist auch denkbar das Deckelteil 12 in der Form einer elastischen Membran auszugestalten, wobei die Spitzen der Kapillaren in dieser Membran ein Loch erzeugen können, das sich nach dem Herausziehen der Kapillaren infolge der Elastizität des Materials der Membran automatisch schließt. Bei einer solchen elastischen Membran wird auch sichergestellt, daß bei der Verriegelung des Aperturhalters 10 am Vorratsgefäß gemäß Figur 5, 6 und 7 der Rand der in der Membran erzeugten Öffnungen dicht an dem Außenumfang der Kapillare 8 anliegt, so daß die zuvor erörterten Dichtungsringe 35 nicht erforderlich sind.

Die Unterteilung in mehrere Teilräume 71 hat den Vorteil, daß gleichzeitig bei Verwendung eines einzigen Vorratsgefäßes 7 mehrere Messungen ausgeführt werden können, wobei der verwendete Aperturhalter 10 durch entsprechende Trennwände 72 ebenfalls in mehrere Blutaufnahmeräume 73 unterteilt ist und wobei jedem dieser Teilräume 73 ein wenigstens eine Apertur 5 aufweisendes Teil 5' und eine eigene Kapillare zugeordnet sind, wie die Figur 11 dies zeigt.

Der Vorteil der Unterteilung des Vorratsgefäßes 7 in mehrere Teilräume 71 besteht darin, daß erstens mehrere Messungen gleichzeitig durchgeführt werden können, was zu einer erheblichen Zeiteinsparung führt und daß zweitens jeder Teilraum 71 weniger Volumen besitzt, so daß bei der Pipetierung derselben Blutmenge die Höhe des Blutes in diesem Raum größer ist als in einem Blutraum ohne Trennwände. Dies hat den Vorteil, daß weniger Blut zur Durchführung einer Messung erforderlich ist und daß die Kapillare nicht soweit bis zum Boden des Vorratsgefäßes 7 geführt werden muß. Vorteilhafterweise kann bei der Messung an Kinderblut, von dem pro Messung häufig nur kleine Blutmengen, z.B. 0,5 ml, zur Verfügung steht, in dem mit Trennwänden 70 versehenen Vorratsgefäß 7 nur ein Teilraum 71 belegt werden, so daß sich auch bei der kleinen zur Verfügung stehenden Blutmenge ein hoher Blutpegel im Teilraum ergibt. In einem vergleichweise großen Vorratsgefäß 7 ohne Trennwände 70 wäre bei einer Blutmenge von 0,5 ml eine Messung nur schwer möglich, weil die Pegelhöhe im Vorratsgefäß sehr gering ist, so daß ein Einsaugen der gesamten im großen Vorratsgefäß verteilten Blutmenge in eine Kapillare kaum möglich wäre.

Am oberen Ende ist der Aperturhalter 10 durch ein Membranteil 76 verschlossen, in das die Verbindungsleitungen zum Zylinder 6 (Figur 1) dicht einführbar sind. Vorzugsweise besteht das Membranteil 76 aus einem Gummi- oder Weichplastikmaterial, in das die Verbindungsleitungen dicht einführbar sind, wobei sich die hierbei entstehenden Löcher beim Entfernen der Verbindungsleitungen aufgrund der Elastizität des Gummi- oder Weichplastikmaterials wieder schließen. Die Trennwand 72 bzw. die Trennwände 72 reichen bis zum Membranteil 76, so daß jeder Blutaufnahmeraum 73 abgedichtet ist. Über die genannten Verbindungsleitungen wird in den Teilräumen ein Unterdruck erzeugt.

In einem Vorratsgefäß mit nur einer Kammer, beispielsweise bei der Vornahme einer Messung, für die nur eine geringe Blutmenge zur Verfügung steht, kann die Pegelhöhe des Blutes im Vorratsgefäß 7 dadurch erhöht werden, daß in das Vorratsgefäß 7 ein Ring 77 eingesetzt wird, der das Volumen des Vorratsgefäßes im unteren Bereich verkleinert.

Wenn dieser Ring 77 aus einem magnetischen Material besteht, kann er auch dazu verwendet werden, das Blut im Vorratsgefäß 7 zu invertieren bzw. zu mischen. Hierzu ist es dann lediglich erforderlich, an der Außenseite des Vorratsgefäßes 7 einen weiteren, vorzugsweise ringförmigen Magneten 78 anzubringen und mit einer Antriebsvorrichtung (nicht dargestellt) in Drehung zu versetzen.

Um eine besonders gute Durchmischung des Blutes im Vorratsgefäß 7 bei einer Drehung des Einsatzringes 77 zu erreichen, kann der Einsatzring 77 an seiner Innenfläche nach innen ragende, vorzugsweise propeller- oder schaufelartige Vorsprünge 79 oder dergleichen aufweisen, die eine Art Rührwerk bilden.

Der Aperturhalter 10 kann in der weiter oben beschriebenen Weise am Vorratsgefäß 7 verriegelbar sein.

## Patentansprüche

1. Einrichtung zur sicheren Entnahme von Blut aus einem Vorratsgefäß (7) mit einem Aperturhalter (10), in dem ein eine Apertur (5) aufweisendes Teil (5') gehalten ist und an dem eine Kapillare (8) befestigt ist, wobei das Vorratsgefäß (7) durch ein Deckelteil (12) verschließbar ist,
dadurch gekennzeichnet,
daß das Deckelteil (12) einen Bereich (16, 17; 14, 15) aufweist, durch den hindurch die Kapillare (8) bei geschlossenem Deckelteil (12) in das Vorratsgefäß (7) einführbar ist und
daß das Vorratsgefäß (7) und der Aperturhalter (10) eine Verriegelungseinrichtung (19, 20; 19, 21) aufweisen, mit deren Hilfe das Vorratsgefäß (7) am Aperturhalter (10) verriegelbar ist.

2. Einrichtung nach Anspruch 1, dadurch gekennzeichnet, daß der Bereich durch eine im Deckelteil (12) befindliche Öffnung (17) gebildet ist, über der sich eine am Deckelteil (12) befestigte, lösbare Folie (16) befindet.

3. Einrichtung nach Anspruch 2, dadurch gekennzeichnet, daß die Folie (16) eine an der dem Aperturhalter (10) zugewandten Seite des Deckelteiles (12) befestigte Adhäsionsfolie ist.

4. Einrichtung nach Anspruch 2 oder 3, dadurch gekennzeichnet, daß die Folie (16) einen über den Randbereich des Deckelteiles (12) vorstehenden Griffbereich (18) aufweist.

5. Einrichtung nach Anspruch 1, dadurch gekennzeichnet, daß der Bereich die Form eines dünnwandigen Gebietes (14) im Deckelteil (12) aufweist, das durch die Kapillare (8) oder ein Spezialwerkzeug durchstoßbar ist.

6. Einrichtung nach Anspruch 5, dadurch gekennzeichnet, daß das Gebiet die Form eines zentralen Bereiches (15') besitzt.

7. Einrichtung nach Anspruch 6, dadurch gekennzeichnet, daß der zentrale Bereich die Form eines Kreuzschlitzes (15) oder eines Kreises (15') aufweist.

8. Einrichtung nach Anspruch 1 bis 7, dadurch gekennzeichnet, daß das Deckelteil (12) mit einem Filmscharnier (13) einteilig und unverlierbar am Vorratsgefäß (7) befestigt ist.

9. Einrichtung nach Anspruch 1 bis 8, dadurch gekennzeichnet, daß die Verriegelungsvorrichtung die Form wenigstens eines (einer) am Aperturhalter (10) angeordneten Vorsprunges (19) (Vertiefung) und wenigstens einer (eines) am Vorratsgefäß (7) vorgesehenen Vertiefung (20, 21) (Vorsprunges) aufweist.

10. Einrichtung nach Anspruch 9, dadurch gekennzeichnet, daß der Vorsprung (19) an einem über die dem Vorratsgefäß (7) zugewandte Seite des Aperturhalters (10) hinausragenden Steg angeordnet ist.

11. Einrichtung nach Anspruch 9 oder 10, dadurch gekennzeichnet, daß über den Umfang des Aperturhalters (10) mehrere Vorsprünge (19) gleichmäßig verteilt sind.

12. Einrichtung nach einem der Ansprüche 9 bis 11, dadurch gekennzeichnet, daß die Vertiefung (21) in der Wandung des Vorratsgefäßes (7) oder in dem diese übergreifenden Teilbereich des Deckelteiles (12) angeordnet ist.

13. Einrichtung nach einem der Ansprüche 9 bis 11, dadurch gekennzeichnet, daß die Vertiefung durch eine Schulter gebildet ist, die im Übergangsbereich zwischen der Wandung des Vorratsgefäßes (7) und dem Deckelteil (12) besteht.

14. Einrichtung nach Anspruch 1 bis 13, dadurch gekennzeichnet, daß die Kapillare (8) und der Aperturhalter (10) einteilig ausgebildet sind.

15. Einrichtung nach Anspruch 1 bis 14, dadurch gekennzeichnet, daß der Aperturhalter (10) an seiner dem Vorratsgefäß (7) abgewandten Seite durch ein Teil (24) verschlossen ist, der eine mittige Öffnung (25) aufweist.

16. Einrichtung nach Anspruch 15, dadurch gekennzeichnet, daß das Teil (24) eine Adhäsionsfolie ist.

17. Einrichtung nach Anspruch 1 bis 16, dadurch gekennzeichnet, daß der Aperturhalter (10) mit der Kapillare (8) und dem daran verriegelten Vorratsgefäß (7) ein Wegwerfteil sind.

18. Einrichtung nach einem der Ansprüche 1 bis 17, dadurch gekennzeichnet, daß das Vorratsgefäß (7) durch wenigstens eine Trennwand (70) in wenigstens zwei Teilräume (71) unterteilt ist.

19. Einrichtung nach Anspruch 18, dadurch gekennzeichnet, daß die Trennwand (70) bis zu dem Deckelteil (12) reicht.

20. Einrichtung nach Anspruch 19, dadurch gekennzeichnet, daß das Deckelteil (12) so gestaltet ist, daß es für jeden Teilraum (71) einen Bereich aufweist, durch den hindurch eine Kapillare (8) bei geschlossenem Deckelteil (12) in den Teilraum (71) einführbar ist.

21. Einrichtung nach einem der Ansprüche 18 bis 20, dadurch gekennzeichnet, daß der Aperturhalter (10) entsprechend dem Vorratsgefäß (7) durch wenigstens eine weitere Trennwand (72) in wenigstens zwei Blutaufnahmeräume (73) unterteilt ist, derart, daß jeweils ein Blutaufnahmeraum (73) des Aperturhalters (10) über eine Apertur eines porösen Teiles (5') und eine Kapillare (8) mit einem Teilraum (71) des Vorratsgefäßes (7) verbindbar ist.

22. Einrichtung nach Anspruch 21, dadurch gekennzeichnet, daß die Trennwand (72) zur Abdichtung der Teilräume (73) bis zu einem Membranteil (76) an der dem Vorratsgefäß abgewandten Seite des Aperturhalters (10) reicht, daß durch das Membranteil (76) eine Verbindungsleitung zur Herstellung eines Unterdrucks in einem entsprechenden Blutaufnahmeraum (73) dicht hindurchführbar ist und daß sich das dabei entstehende Loch aufgrund der Elastizität des Membranteiles (76) nach Entfernen der Verbindungsleitung wieder schließt.

23. Einrichtung nach Anspruch 22, dadurch gekennzeichnet, daß das Membranteil (76) aus einem Gummi- oder Weichplastikmaterial besteht.

24. Einrichtung nach einem der Ansprüche 18 bis 23, dadurch gekennzeichnet, daß das Vorratsgefäß (7) in zwei oder vier Teilräume (71) unterteilt ist.

25. Einrichtung nach einem der Ansprüche 1 bis 24, dadurch gekennzeichnet, daß ein das Volumen des Vorratsgefäßes (7) verkleinernder Einsatzring (77) in das Vorratsgefäß (7) oder in einem Teilraum (71) desselben einsetzbar ist.

26. Einrichtung nach Anspruch 25, dadurch gekennzeichnet, daß der Einsatzring (77) im Vorratsgefäß drehbar angeordnet ist.

27. Einrichtung nach Anspruch 26, dadurch gekennzeichnet, daß der Einsatzring (77) aus einem magnetischen Material besteht und daß eine das Vorratsgefäß (7) außen im Bereich des Einsatzringes (77) umgebende, drehbare Magneteinrichtung vorgesehen ist.

28. Einrichtung nach Anspruch 27, dadurch gekennzeichnet, daß die Magneteinrichtung ein ringförmiger Magnet (78) ist.

29. Einrichtung nach einem der Ansprüche 25 bis 28, dadurch gekennzeichnet, daß der Einsatzring (77) an seiner Innenfläche Vorsprünge (79) aufweist, durch die bei der Drehung des Einsatzringes (77) die Durchmischung des im Vorratsgefäß (7) bzw. im Teilraum (71) enthaltenen Blutes gefördert wird.

## Claims

1. A device for reliably removing blood from a supply vessel (7) having an aperture holder (10) which holds a part (5') comprising an aperture (5) and to which aperture holder is attached a capillary (8), wherein the supply vessel (7) can be closed by means of a cover part (12), characterised in that the cover part (12) comprises a region (16, 17; 14, 15), through which the capillary (8) can be inserted into the supply vessel (7) when the cover part (12) is closed and that the supply vessel (7) and the aperture holder (10) comprise a locking device (19, 20: 19, 21) with the aid of which it is possible to lock the supply vessel (7) to the aperture holder (10).

2. A device according to claim 1, characterised in that the region is formed by an opening (17) located in the cover part (12) and located above this opening is a detachable sheet (16) which is attached to the cover part (12).

3. A device according to claim 2, characterised in that the sheet (16) is an adhesive sheet attached at the side of the cover part (12) facing the aperture holder (10).

4. A device according to claim 2 or 3, characterised in that the sheet (16) comprises a grip region (18) which protrudes beyond the edge region of the cover part (12).

5. A device according to claim 1, characterised in that the region is in the form of a thin-walled area ((14) in the cover part (12), which thin-walled area can be penetrated by the capillary (8) or a special tool.

6. A device according to claim 5, characterised in that the area is in the form of a central region (15').

7. A device according to claim 6, characterised in that the central region is in the form of a crossed slot (15 or a circle (15').

8. A device according to claim 1 to 7, characterised in that the cover part (12) is attached by means of a sheet hinge (13) to the supply vessel (7) as one piece and in such a manner that it cannot become lost.

9. A device according to claim 1 to 8, characterised in that the locking device is in the form at least of one projection (19) (recess) disposed on the aperture holder (10) and at least one recess (20, 21) (projection) provided in the supply vessel (7).

10. A device according to claim 9, characterised in that the projection (19) is disposed on a web protruding beyond the side of the aperture holder (10) facing the supply vessel (7).

11. A device according to claim 9 or 10, characterised in that the a plurality of projections (19) are distributed uniformly over the periphery of the aperture holder (10).

12. A device according to any one of claims 9 to 11, characterised in that the recess (21) is disposed in the wall of the supply vessel (7) or in the partial region of the cover part (12) which laps over the said wall.

13. A device according to any one of claims 9 to 11, characterised in that the recess is formed by means of a shoulder which exists in the transition region between the wall of the supply vessel (7) and the cover part (12).

14. A device according to claim 1 to 13, characterised in that the capillary (8) and the aperture holder (10) are formed as one piece.

15. A device according to claim 1 to 14, characterised in that the aperture holder (10) is closed on its side remote from the supply vessel (7) by means of a part (24) which comprises a central opening (25).

16. A device according to claim 15, characterised in that the part (24) is an adhesive sheet.

17. A device according to claim 1 to 16, characterised in that the aperture holder (10) having the capillary (8) and the supply vessel (7) locked thereto are a disposable part.

18. A device according to any one of claims 1 to 17, characterised in that the supply vessel (7) is subdivided by means of at least one partitioning wall (70) into at least two sub-chambers (71).

19. A device according to claim 18, characterised in that the partitioning wall (70) extends as far as the cover part (12).

20. A device according to claim 19, characterised in that the cover part (12) is designed such that it comprises for each sub-chamber (71) a region through which a capillary (8) can be inserted into the sub-chamber (71) when the cover part (12) is closed.

21. A device according to any one of claims 18 to 20, characterised in that the aperture holder (10) is subdivided corresponding to the supply vessel (7) by means of at least a further partitioning wall (72) into at least two blood receiving chambers (73), in such a manner that in each case one blood receiving chamber (73) of the aperture holder (10) can be connected by way of an aperture of a porous part (5') and a capillary (8) to a sub-chamber (71) of the supply vessel (7).

22. A device according to claim 21, characterised in that the partitioning wall (72) for the purpose of sealing the sub-chambers (73) extends as far as one membrane part (76) at the side of the aperture holder (10) remote from the supply vessel, that a connecting line for the purpose of producing a negative pressure in a corresponding blood receiving chamber (73) can be inserted in a sealed manner through the membrane part (76) and that the ensuing hole closes again after the removal of the connecting line owing to the elasticity of the membrane part (76).

23. A device according to claim 22, characterised in that the membrane part (76) consists of a rubber or soft plastic material.

24. A device according to any one of claims 18 to 23, characterised in that the supply vessel (7) is subdivided into two or four sub-chambers (71).

25. A device according to any one of claims 1 to 24, characterised in that an insert ring (77) which reduces the volume of the supply vessel (7) can be inserted into the supply vessel (7) or in a sub-chamber (71) thereof.

26. A device according to claim 25, characterised in that the insert ring (77) is rotatably disposed in the supply vessel.

27. A device according to claim 26, characterised in that the insert ring (77) consists of a magnetic material and that a rotatable magnet device is provided which surrounds the supply vessel (7) outside in the region of the insert ring (77).

28. A device according to claim 27, characterised in that the magnet device is an annular magnet (78).

29. A device according to any one of claims 25 to 28, characterised in that the insert ring (77) comprises on its inner surface projections (79) which in the case of the insert ring (77) being rotated promote the mixing of the blood contained in the supply vessel (7) and in the sub-chamber (71).

## Revendications

1. Appareil pour prélever sans risque le sang d'un récipient de stockage (7) avec un support à ouverture (10) dans lequel est maintenue une pièce (5') présentant une ouverture (5) et sur lequel est fixé un tube capillaire (8), le récipient de stockage (7) pouvant être fermé par une partie couvercle (12), caractérisé en ce que
la partie couvercle (12) présente une zone (16, 17; 14, 15) par laquelle le tube capillaire (8) peut être introduit dans le récipient de stockage (7) avec la partie couvercle (7) fermée et
en ce que le récipient de stockage (7) et le support avec ouverture (10) présentent un dispositif de verrouillage (19, 20; 19, 21) à l'aide duquel le récipient de stockage (7) peut être verrouillé sur le support avec ouverture (10).

2. Appareil selon la revendication 1, caractérisé en ce que la zone est constituée par un orifice (17) se trouvant dans la partie couvercle (12) au-dessus duquel se trouve une feuille (16) détachable et fixée sur la partie couvercle (12).

3. Appareil selon la revendication 2, caractérisé en ce que la feuille (16) est une feuille à adhérence fixée sur le côté de la partie couvercle (12) tourné vers le support avec ouverture (10).

4. Appareil selon la revendication 2 ou 3, caractérisé en ce que la feuille (16) présente une zone de prise (18) qui déborde de la zone périphérique de la partie couvercle (12).

5. Appareil selon la revendication 1, caractérisé en ce que la zone a la forme d'une région à paroi mince (14) dans la partie couvercle (12) que l'on peut passer à travers le tube capillaire (8) ou un outil spécial.

6. Appareil selon la revendication 5, caractérisé en ce que la région a la forme d'une zone centrale (15').

7. Appareil selon la revendication 6, caractérisé en ce que la zone centrale a la forme d'une empreinte cruciforme (15) ou d'un cercle (15').

8. Appareil selon les revendications 1 à 7, caractérisé en ce que la partie couvercle (12) est fixée de façon imperdable avec une charnière à film (13) sur le réservoir de stockage (7), en formant un seul bloc.

9. Appareil selon les revendications 1 à 8, caractérisé en ce que le dispositif de verrouillage a la forme d'au moins une partie en saillie (19) (cavité) disposée sur le support avec ouverture (10) et d'au moins une cavité (20, 21) (partie en saillie) prévue sur le réservoir de stockage (7).

10. Appareil selon la revendication 9, caractérisé en ce que la partie en saillie (19) est disposée sur une barrette dépassant du côté du support avec ouverture (10) tourné vers le récipient de stockage (7).

11. Appareil selon la revendication 9 ou 10, caractérisé en ce que plusieurs parties en saillie (19) sont réparties uniformément à la périphérie du support avec ouverture (10).

12. Appareil selon l'une des revendications 9 à 11, caractérisé en ce que la cavité (21) est disposée dans la paroi du réservoir de stockage (7) ou dans la zone partielle la recouvrant de la partie couvercle (12).

13. Appareil selon l'une des revendications 9 à 11, caractérisé en ce que la cavité est constituée par un épaulement qui consiste dans la zone de transition entre la paroi du réservoir de stockage (7) et la partie couvercle (12).

14. Appareil selon les revendications 1 à 13, caractérisé en ce que le tube capillaire (8) et le support avec ouverture (10) sont constitués d'une seule pièce.

15. Appareil selon les revendications 1 à 14, caractérisé en ce que le support avec ouverture (10) est fermé sur son côté opposé au récipient de stockage (7) par une pièce qui présente une ouverture centrée (25).

16. Appareil selon la revendication 15, caractérisé en ce que la pièce (24) est une feuille à adhérence.

17. Appareil selon les revendications 1 à 16, caractérisé en ce que le support avec ouverture (10) avec le tube capillaire (8) et le récipient de stockage (7) verrouillé dessus sont une pièce jetable.

18. Appareil selon l'une des revendications 1 à 17, caractérisé en ce que le récipient de stockage (7) est subdivisé par au moins une cloison de séparation (70) en au moins deux compartiments partiels (71).

19. Appareil selon la revendication 18, caractérisé en ce que la cloison de séparation (70) va jusqu'à la partie couvercle (12).

20. Appareil selon la revendication 19, caractérisé en ce que la partie couvercle (12) est conçue de façon qu'elle présente pour chaque compartiment partiel (71) une zone par laquelle on peut introduire un tube capillaire (8) avec la partie couvercle fermée (12) dans le compartiment partiel (71).

21. Dispositif selon l'une des revendications 18 à 20, caractérisé en ce que le support avec ouverture (10) est subdivisé conformément au récipient de stockage (7) par au moins une autre cloison de séparation (72) en au moins deux compartiments de réception de sang (73) de telle façon que, à chaque fois, un compartiment de réception du sang (73) du support avec ouverture (10) puisse être relié par une ouverture d'une partie poreuse (5') et un tube capillaire (8) avec un compartiment partiel (71) du récipient de réserve (7).

22. Appareil selon la revendication 21, caractérisé en ce que, pour l'étanchéité des compartiments partiels (73), la cloison de réception (72) va jusqu'à une membrane (76) sur le côté du support avec ouverture (10) opposé au récipient de stockage, en ce que, par la membrane (76), on peut faire passer de façon étanche une conduite de liaison pour l'établissement d'une dépression dans un compartiment de réception du sang et en ce que le trou qui se forme ainsi se referme du fait de l'élasticité de la membrane (76) après l'enlèvement de la conduite de liaison.

23. Appareil selon la revendication 22, caractérisé en ce que la membrane (76) est composée d'un matériau de caoutchouc ou de plastique mou.

24. Appareil selon l'une des revendications 18 à 23, caractérisé en ce que le récipient de stockage (7) est subdivisé en deux ou quatre compartiments partiels (71).

25. Appareil selon l'une des revendications 1 à 24, caractérisé en ce qu'une bague à insertion (77) réduisant le volume du récipient de stockage (7) peut être insérée dans le récipient de stockage (7) ou dans un compartiment partiel (71).

26. Appareil selon la revendication 25, caractérisé en ce que la bague à insertion (77) est disposée dans le récipient de stockage de façon à pouvoir tourner.

27. Appareil selon la revendication 26, caractérisé en ce que la bague à insertion (77) est dans un matériau magnétique et qu'il est prévu un dispositif magnétique tournant et entourant le récipient de stockage (7) à l'extérieur dans la zone de la bague à insertion (77).

28. Appareil selon la revendication 27, caractérisée en ce que le dispositif magnétique est un aimant (78) de forme annulaire.

29. Appareil selon l'une des revendications 25 à 28, caractérisé en ce que la bague d'insertion (77) prrésente sur sa surface intérieure des parties en saillie (79) par lesquelles le mélange du sang contenu dans le récipient de stockage (7) ou dans le compartiment partiel (71) est transporté lors de la rotation de la bague à insertion (77).
